(19) Europäisches Patentamt
European Patent Office
Office européen des brevets

(11) Publication number: **0 495 313 A1**

# (12) EUROPEAN PATENT APPLICATION

(21) Application number: **91311935.0**

(51) Int. Cl.$^5$: **C07C 231/02**, C07C 233/25

(22) Date of filing: **23.12.91**

(30) Priority: **16.01.91 JP 14910/91**

(43) Date of publication of application:
**22.07.92 Bulletin 92/30**

(84) Designated Contracting States:
**DE FR GB NL**

(71) Applicant: **KONICA CORPORATION**
**26-2, Nishishinjuku 1-chome, Shinjuku-ku**
**Tokyo 160(JP)**

(72) Inventor: **Uchida, Takashi, c/o Konica Corporation**
**No. 1, Sakura-machi, Hino-shi**
**Tokyo 191(JP)**
Inventor: **Mizukura, Noboru, c/o Konica Corporation**
**No. 1, Sakura-machi, Hino-shi**
**Tokyo 191(JP)**

(74) Representative: **Brock, Peter William et al**
**UROUHART-DYKES & LORD 91 Wimpole Street**
**London W1M 8AH(GB)**

## (54) Process for preparing acylamino compounds.

(57) There is provided a process for preparing an acylamino compound of the general formula[I]:

R'
|
Ar – NCOR [I]

wherein Ar is aryl or a heterocyclic group, R is an alkyl group having at least one fluoro-substituent on the carbon atom adjacent to the carbonyl carbon, and R' is hydrogen, alkyl, aryl or a heterocyclic group, which comprises reacting a compound of the general formula[II]:

R'
|
Ar – NH [II]

wherein Ar and R' are as defined above, with a compound of the general formula[III]:

R – COOH [III]

wherein R is as defined above, in the presence of a base and thionyl chloride.

EP 0 495 313 A1

[BACKGROUND OF THE INVENTION]

## Field of the Invention

The present invention relates to a process for preparing acylamino compounds useful for the production of medicaments, dyes, synthetic fibers, photographic materials, etc.

## Prior Art

Acylamino compounds have hitherto been prepared by reacting an amine with an acid chloride or by reacting an amine with an acid anhydride (see, for example, R.B. Wagner, H.D. Zook, 1953, SYNTHETIC ORGANIC CHEMISTRY, pages 565-589, Wiley, New York). Such methods require additional step of synthesizing an acid chloride or an acid anhydride from a carboxylic acid. Furthermore, in the method of using an acid anhydride for the preparation of acylamino compounds, one molar carboxylic acid is wasted per mol of acid anhydride. Therefore, such method is not suitable for the preparation of acylamino compounds if the starting carboxylic acid is relatively expensive, for example, those having fluoro-substituent(s). In addition, acid chlorides and acid anhydrides are generally unstable.

J. F. Normant, H. Deshayes, Bull. Soc. Chim. Fr., 1972, 2854, discloses a process for preparing acylamino compounds by reacting a carboxylic acid with an amine in the presence of thionyl chloride. The yield of this process, however, is considerably low, as clearly shown hereinafter in Comparative Example 1.

Japanese Published Unexamined Patent Application No. 225635/1989 describes that acylamino compounds can be obtained merely by mixing a carboxylic acid having fluoro-substituent(s) on the carbon atom adjacent to the carbonyl carbon, with an amine. Though this method produces an acylamino compound from an aliphatic amine and fluoro-substituted carboxylic acid, it could not produce an acylamine compound successfully from an aromatic amine and fluoro-substituted carboxylic acid.

## Provblems to be solved by the Invention

Therefore, there is a need for a process for preparing acylamino compounds directly and in high yield from a carboxylic acid having fluoro-substituent(s) on the carbon atom adjacent to the carbonyl carbon, and an arylamine or a heterocyclic amine.

## Means for solving the Problem

It has surprisingly been found that the above object can effectively be attained by reacting an amine with a carboxylic acid in the presence of a base and thionyl chloride.

[SUMMARY OF THE INVENTION]

Thus, the present invention relates to a process for preparing an acylamino compound of the general formula[I]:

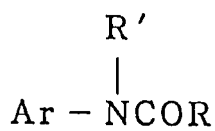

[I]

wherein Ar is aryl or a heterocyclic group, R is an alkyl group having at least one fluoro-substituent on the carbon atom adjacent to the carbonyl carbon, and R' is hydrogen, alkyl, aryl or a heterocyclic group, which comprises reacting a compound of the general formula[II]:

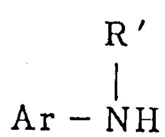

[II]

wherein Ar and R' are as defined above, with a compound of the general formula[III]:

$$R-COOH \qquad [III]$$

wherein R is as defined above, in the presence of a base and thionyl chloride.

[DETAILED DESCRIPTION OF THE INVENTION]

Suitable examples of aryls in the definition of Ar are phenyl and naphthyl. Suitable examples of the heterocyclic groups in the definition of Ar include pyridyl, pyrimidinyl, quinolyl, imidazolyl and furyl.

Alkyl groups as defined above having at least one fluoro-substituent on the carbon atom adjacent to the carbonyl carbon include straight, branched and unsaturated alkyls, for example, $CF_3$-, $CClF_2$-, $CHF_2$-, $C_2F_5$-, $CF_3CHF$-, $CFBr_2$-, $C_3F_7$-, $C_7F_{15}$-, $H(CF_2)_3$-, $CF_3(CF_2)_{10}$-, $H(CF_2)_8$-, $Cl(CF_2)_8$-, $CF_2=CFCF_2$-,$F[CF(CF_3)$-$CF_2O]_5CF(CF_3)$-, $C_3F_7OCF(CF_3)$- and $C_2H_5OCO(CF_2)_3$-.

Alkyls in the definition of R' include straight, branched and cyclic alkyls having 1-22 carbon atoms, for example, methyl, ethyl, isopropyl, pentyl, 2-ethylhexyl and cyclohexyl.

Aryl in the definition of R' is preferably phenyl.

Examples of heterocyclic groups in the definition of R' are the same as those as mentioned in Ar.

Ar and R' may have additional substituents.

Suitable bases which may be mentioned include organic bases such as pyridine, N,N-dimethylaniline, 4-N,N-dimethylaminopyridine, triethylamine, N,N-diisopropylethylamine, hexamethylenetetramine, 1, 5-diazabicyclo[3. 4. 0]nonene-5, imidazole, trioctylamine, quinoline, picoline and collidine.

Basic ion exchange resins can be used as a base in the present invention, either alone or in combinations thereof.

The process of the present invention may be conducted without any solvent, but preferably it may be conducted in the presence of an organic solvent conventionally used for acylation. Examples of such solvents are acetonitrile, ethyl acetate, chloroform, dioxane, tetrahydrofuran, methyl cellosolve, toluene, ether, and the like.

The process of the present invention may be facilitated by the addition of a catalytic amount of N,N-dimethylformamide.

The amount of the base used in the present invention is above 0.5 mol, and generally in the range of 1-2 mol, per mol of the starting compound[III].

The bases as mentioned above may also be used as a solvent in the process of the present invention. In such case, they are used in large excess.

Though the reaction of the present invention can be carried out at an ambient temperature, it may also be performed at an elevated temperature preferably ranging from 40°C to 100°C, in order to facilitate the reaction.

The order of the addition of the reactants is not critical, but it is preferred to prepare first a mixture of an amine, a carboxylic acid and a base, and then add thionyl chloride dropwise. The temperature of the mixture during the addition of thionyl chloride is preferably maintained above room temperature, though the reaction also progresses even under cooling.

The amount of thionyl chloride added may be equimolar relative to the amount of the compound [III], or preferably in slight excess thereof.

According to the invention, acylamino compounds can be obtained easily and in high yield in one step.

Several non-limiting examples of the compounds which may be prepared according to the present invention are as follows:

1

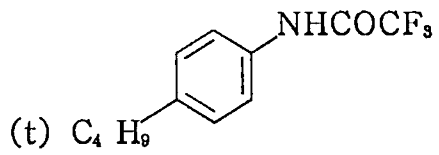

2

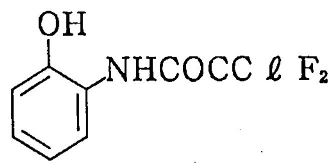

3

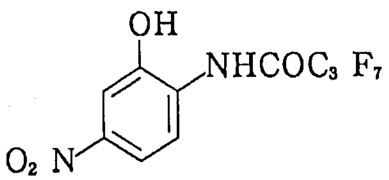

4

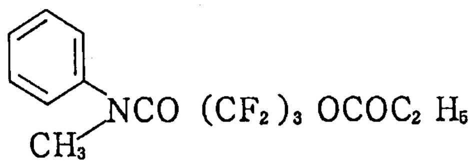

5

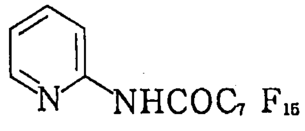

6

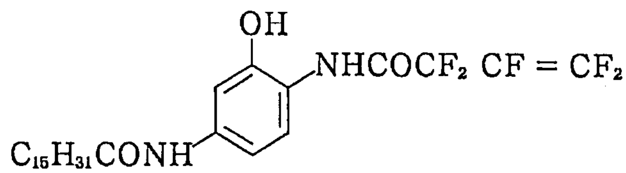

7

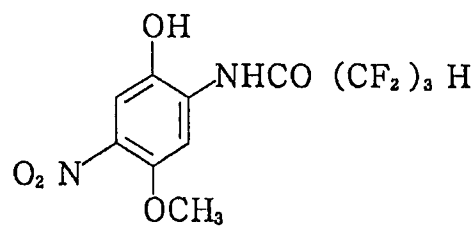

8

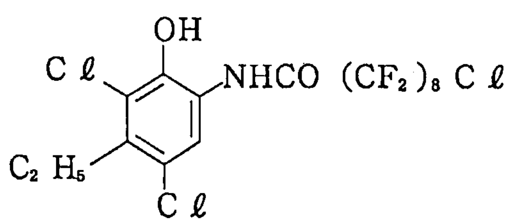

9

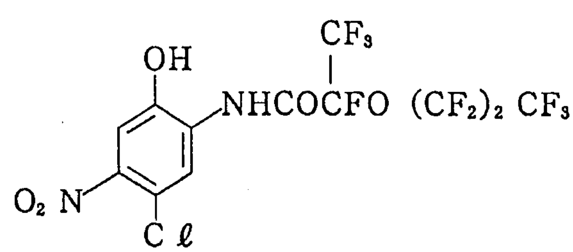

10

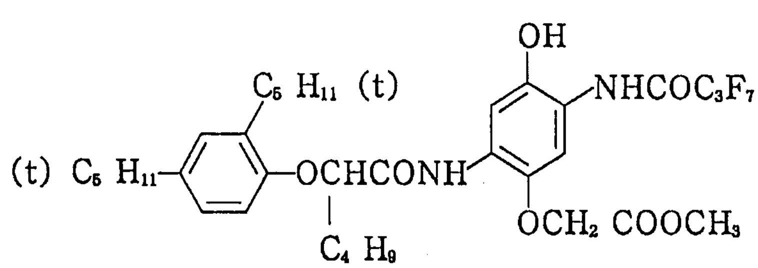

The present ivention is further illustrated by the following Example which in no way limits the invention.

Example:

Preparation of 2-heptafluorobutyrylamino-5-nitrophenol (Compound No. 3)

77g of 5-nitro-2-aminophenol was dissolved in a mixture of 350ml of acetonitrile and 40ml of pyridine. To the resultant solution, 3.0ml of N,N-dimethylformamide was added. Then, 112g of heptafluorobutyric acid was added while stirring at room temperature. Slightly exothermic reaction took place without adversely affecting the present process. The mixture thus obtaind was heated to about 50 ° C, and 62.5g of thionyl chloride was added dropwise at such rate as maintaining gentle reflux of the reaction mixture (A slightly exothermic reaction.). After the addition of thionyl chloride was complete, the reaction mixture was refluxed with heating for further 30 minutes. Then, the solvent was removed from the mixture under reduced pressure, the residue was taken up into an ice-water containing hydrochloric acid, and the resultant mixture was stirred well to give crystals which were removed by filtration. The crystals thus obtained were thoroughly washed with water and recrystallized from 80% methanol to give the title compound in 93.5% yield. m.p. 146-7 ° C.

From the filtrate, pyridine was recovered by extracting with organic solvent after adding excess amount of sodium hydroxide.

Comparative Example 1:

(The method as described by J. F. Normant, H. Deshayes, in Bull. Soc. Chim. Fr., 1972, 2854)

The procedure of Example 1 was repeated without using pyridine. The yield was 43%.

Comparative Example 2:

(Method as described in the Japanese Published Unexamined Patent Application No. 225635/1989)

7.7g of 5-nitro-2-aminophenol was dissolved in 50ml of N,N-dimethylformamide. To the solution, 11.2g of heptafluorobutyric acid was added, and the resultant mixture was stirred. However, no reaction occurred either at room temperature or at an elevated temperature.

## Claims

1. A process for preparing an acylamino compound of the general formula[I]:

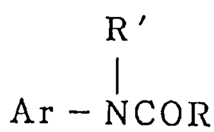

[I]

wherein Ar is aryl or a heterocyclic group, R is an alkyl group having at least one fluoro-substituent on the carbon atom adjacent to the carbonyl carbon, and R' is hydrogen, alkyl, aryl or a heterocyclic group, which comprises reacting a compound of the general formula[II]:

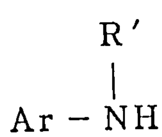

[II]

wherein Ar and R' are as defined above, with a compound of the general formula[III]:

$$R-COOH \qquad [III]$$

wherein R is as defined above, in the presence of a base and thionyl chloride.

2. A process according to claim 1 wherein Ar is 2-hydroxy-4-nitrophenyl.

3. A process according to claim 1 wherein R is polyfluoro-substituted alkyl.

**4.** A process according to claim 3 wherein R is heptafluoropropyl.

**5.** A process according to claim 1 wherein R' is hydrogen.

European Patent Office

# EUROPEAN SEARCH REPORT

Application Number

EP 91311935.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE - C - 175 586 (ALFRED VON JANSON) * Totality * | 1,5 | C 07 C 231/02<br>C 07 C 233/25 |
| A | GB - A - 1 065 801 (NIPPON SODA K.K.) * Examples II,IV * | 1 | |
| A<br>D | PATENT ABSTRACTS OF JAPAN, unexamined applications, C field, vol. 13, no. 549, December 7, 1989 THE PATENT OFFICE JAPANESE GOVERNMENT page 65 C 662 * Kokai-no. 1-225 635 (MITSUBISHI ELECTRIC CORP.) * | 1,5 | |
| A | DE - A - 1 966 974 (PALOMO COLL) * Example 4 * | 1,3,5 | TECHNICAL FIELDS SEARCHED (Int. Cl.5) |
| D,A | SOCIETE CHIMIQUE DE FRANCE, no. 7, 1972, JEAN F. NORMANT et al. "Utilisation du chlorure de thionyle dans l'hexamethyl-phosphoramide" pages 2854-2859 * Page 2858, right column, lines 1-7 from the bottom * | 1,5 | C 07 C 231/00<br>C 07 C 233/00 |
| A | REAKTIONEN DER ORGANISCHEN SYNTHESE, 1978, Georg Thieme Verlag, Stuttgart, CESARE FERRI "Reaktionen der organischen Synthese" page 221 * Reaction 6 * | 1 | |

The present search report has been drawn up for all claims

| Place of search | Date of completion of the search | Examiner |
|---|---|---|
| VIENNA | 22-04-1992 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)